# EUROPÄISCHE PATENTSCHRIFT

(11) **EP 2 239 258 B1**
(45) Veröffentlichungstag und Bekanntmachung des Hinweises auf die Patenterteilung: **14.03.2012**
(21) Anmeldenummer: 10167054.5
(22) Anmeldetag: 18.07.2006
(51) Int. Cl.: C07D 265/16, C07D 498/04, C10L 1/22, C09K 15/20, C10M 133/48

(54) **Tetrahydrobenzoxazine**
Tetrahydrobenzoxazines
Tetrahydrobenzoxazines

(30) Priorität: 26.07.2005 DE 102005035527
(43) Veröffentlichungstag der Anmeldung: 13.10.2010
(62) Teilanmeldung aus: 06777819.1
(73) Patentinhaber: BASF SE, 67056 Ludwigshafen (DE)
(72) Erfinder: Lange, Arno, 67098, Bad Dürkheim (DE); Mach, Helmut, 69115, Heidelberg (DE); Rath, Hans Peter, 67269, Grünstadt (DE); Posselt, Dietmar, 69120, Heidelberg (DE)

(56) Entgegenhaltungen:
- EP-A1- 0 831 141
- WO-A-01/25293
- WO-A2-03/106595
- JP-A- 63 149 646
- US-A- 2 458 526
- US-A- 2 459 112
- US-A- 4 207 104

## Beschreibung

Die vorliegende Erfindung betrifft spezielle Tetrahydrobenzoxazine mit einem oder mehreren längerkettigen Hydrocarbylresten, welche als Stabilisatoren zur Stabilisierung von unbelebtem organischen Material gegen die Einwirkung von Licht, Sauerstoff und Wärme, insbesondere in Turbinenkraftstoffen (jet fuels), geeignet sind.

Die mechanischen, chemischen und/oder ästhetischen Eigenschaften von unbelebtem organischen Material, z. B. von Kunststoffen und Lacken, aber auch von Mineralölprodukten und Kraftstoffen, werden bekanntermaßen durch die Einwirkung von Licht, Sauerstoff und Wärme verschlechtert. Diese Verschlechterung zeigt sich üblicherweise als Vergilbung, Verfärbung, Rissbildung oder Versprödung des Materials. Es sind schon Stabilisatoren oder Stabilisatorzusammensetzungen bekannt, mit denen ein verbesserter Schutz gegen eine solche Beeinträchtigung von organischem Material durch Licht, Sauerstoff und Wärme erzielt werden kann.

So werden in der WO 05/073152 (1) 2-Alkyl-polyisobutenylphenole und deren Mannich-Addukte als Antioxidantien zur Stabilisierung von unbelebtem organischen Material gegen die Einwirkung von Licht, Sauerstoff und Wärme beschrieben. Als zu stabilisierende Materialien werden auch Kraftstoffe wie Ottokraftstoffe, Dieselkraftstoffe und Turbinenkraftstoffe sowie Schmierstoffzusammensetzungen genannt. In Turbinenkraftstoffen bewirken diese 2-Alkyl-polyisobutenyl-phenole und deren Mannich-Addukte eine Verbesserung der Thermostabilität sowie eine Verringerung der Ablagerungen im Kraftstoffkreislauf und Verbrennungssystem der Turbinen.

US-A-2459112 offenbart ebenfalls strukturell unterschiedliche Tetrahydrobenzoxazinderivate als Antioxidantien zur stabilisierung von Mineralölen.

Auch die WO 03/106595 (2) offenbart neben hydrocarbylsubstituierten Bernsteinsäurederivaten und Polyalkenylthiophosphonatestern Mannich-Addukte aus hydrocarbylsubstituierten Phenolen, einem Aldehyd und einem Amin als Additive für Turbinenkraftstoffe (jet fuels) zur Verbesserung der Thermostabilität sowie zur Verringerung von Ablagerungen.

Es besteht jedoch, insbesondere für den Mineralölprodukte- und Kraftstoff-Bereich, ein Bedarf an Stabilisatoren bzw. Antioxidantien mit verbesserter Schutzwirkung gegen die Beeinträchtigung der Materialeigenschaften durch Licht, Sauerstoff und Wärme. Vor allem für Turbinenkraftstoffe (jet fuels), die einer extremen thermischen Belastung beim und vor dem Verbrennungsvorgang in Turbinen, beispielsweise in Flugzeugturbinen, ausgesetzt sind, werden neue verbesserte Stabilisatoren gesucht. Diese sollen in den Turbinen gleichzeitig über ihre Wirkungsweise als Antioxidantien und/oder Dispergatoren auch Ablagerungen im Kraftstoffkreislauf und im Verbrennungssystem verringern.

Es bestand daher die Aufgabe, Stabilisatoren mit einer verbesserten Stabilisierung von unbelebtem organischen Material, insbesondere von Mineralölprodukten und Kraftstoffen, vor allem von Turbinenkraftstoff, gegen die Einwirkung von Licht, Sauerstoff und Wärme bereitzustellen.

Spezielle Tetrahydrobenzoxazine, bei denen sich ein oder mehrere längerkettige Hydrocarbylreste mit jeweils 25 bis 3000 Kohlenstoffatomen als Substituenten an den Stickstoffatomen der Tetrahydrooxazin-Ringe befinden, sind neu. Daher sind Gegenstand der vorliegenden Erfindung Tetrahydrobenzoxazine der allgemeinen Formel la

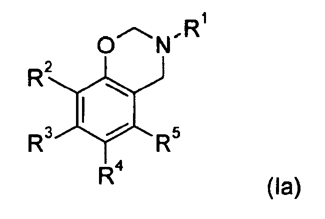

in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können,
wobei der Substituent R⁴ auch für einen Rest der Formel Y stehen kann 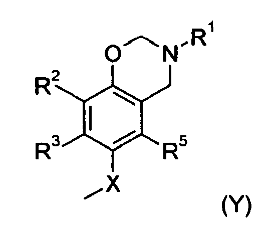 in dem die Substituenten R¹, R², R³ und R⁵ die vorgenannten Bedeutungen haben und der Substituent X ein Kohlenwasserstoff-Brückenglied bezeichnet, welches aus einem oder mehreren Isobuten-Einheiten besteht oder ein oder mehrere Isobuten-Einheiten enthält, oder
wobei der Substituent R⁴ auch für einen Rest der Formel Z oder Z' stehen kann 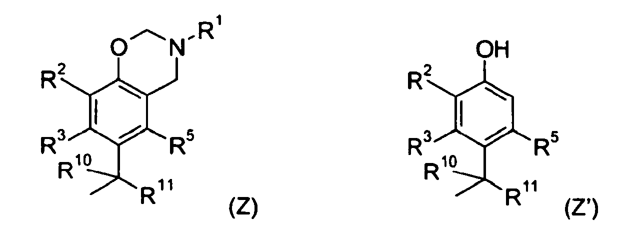 in denen die Substituenten R¹, R², R³ und R⁵ die vorgenannten Bedeutungen haben und die Substituenten R¹⁰ und R¹¹ gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR⁷-CH₂- auch einen zweiten Tetrahydrooxazin-Ring oder die Substituenten R² und R³ und R⁴ und R⁵ mit den an den Benzolkern angebundenen Teilstrukturen -O-CH₂-NR⁷-CH₂- und -O-CH₂-NR⁸-CH₂- auch einen zweiten und einen dritten Tetrahydrooxazin-Ring ausbilden können,
wobei R⁷ und R⁸ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können,
mit der Maßgabe, dass mindestens einer der Substituenten R¹ oder R⁷ oder R⁸ 25 bis 3000 Kohlenstoffatome aufweist und die übrigen Substituenten aus der Gruppe R¹, R², R3, R⁴, R⁵, R⁷ und R⁸, wenn sie für Hydrocarbylreste stehen, jeweils 1 bis 20 Kohlenstoffatome aufweisen.

Spezielle Tetrahydrobenzoxazine mit Hydroxylgruppen, die am Benzolkern in 2- und/oder 4-Position stehen, und mit längerkettigen Hydrocarbylresten mit jeweils 16 bis 3000 Kohlenstoffatomen als Substituenten an den Stickstoffatomen der Tetrahydrooxazin-Ringe sind neu. Daher sind auch Gegenstand der vorliegenden Erfindung Tetrahydrobenzoxazine der allgemeinen Formel Ib 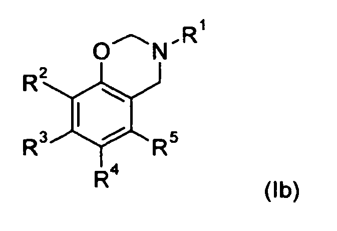 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet, wobei R⁶ ein Wasserstoffatom oder einen C₁- bis C₄-Alkylrest bezeichnet, und
die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können,
wobei der Substituent R⁴ auch für einen Rest der Formel Y stehen kann 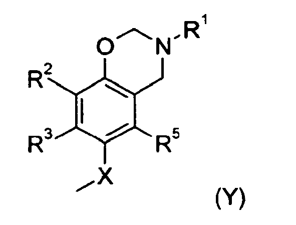 in dem die Substituenten R¹, R², R³ und R⁵ die vorgenannten Bedeutungen haben und der Substituent X ein Kohlenwasserstoff-Brückenglied bezeichnet, welches aus einem oder mehreren Isobuten-Einheiten besteht oder ein oder mehrere Isobuten-Einheiten enthält, oder
wobei der Substituent R⁴ auch für einen Rest der Formel Z oder Z' stehen kann 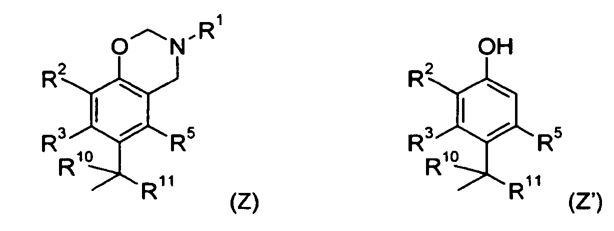 in denen die Substituenten R¹, R², R³ und R⁵ die vorgenannten Bedeutungen haben und die Substituenten R¹⁰ und R¹¹ gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR⁷-CH₂- auch einen zweiten Tetrahydrooxazin-Ring ausbilden können,
wobei R⁷ einen Hydrocarbylrest mit 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein kann,
mit der Maßgabe, dass mindestens einer der Substituenten R¹ oder R⁷ 16 bis 3000 Kohlenstoffatome aufweist und die übrigen Substituenten aus der Gruppe R¹, R², R³, R⁴, R⁵ und R⁷, wenn sie für Hydrocarbylreste stehen, jeweils 1 bis 20 Kohlenstoffatome aufweisen,
und mit der Maßgabe, dass die Substituenten R² und/oder R⁴ für Hydroxylgruppen stehen.

Spezielle Tetrahydrobenzoxazine, die einen zweiten oder einen zweiten und einen dritten Tetrahydrooxazin-Ring am Benzolkern aufweisen, sind neu. Daher sind auch Gegenstand der vorliegenden Erfindung Tetrahydrobenzoxazine der allgemeinen Formel Id 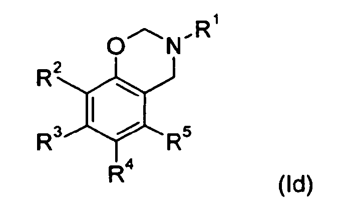 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können,
wobei der Substituent R⁴ auch für einen Rest der Formel Y stehen kann 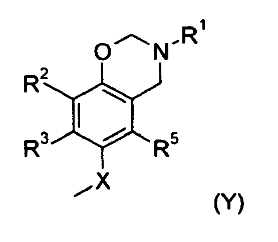 in dem die Substituenten R¹, R², R³ und R⁵ die vorgenannten Bedeutungen haben und der Substituent X ein Kohlenwasserstoff-Brückenglied bezeichnet, welches aus einem oder mehreren Isobuten-Einheiten besteht oder ein oder mehrere Isobuten-Einheiten enthält, oder
wobei der Substituent R⁴ auch für einen Rest der Formel Z oder Z' stehen kann 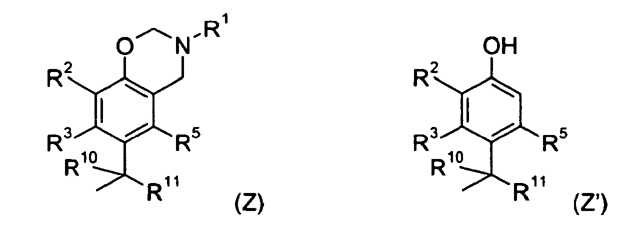 in denen die Substituenten R¹, R², R³ und R⁵ die vorgenannten Bedeutungen haben und die Substituenten R¹⁰ und R¹¹ gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR⁷-CH₂- einen zweiten Tetrahydrooxazin-Ring oder die Substituenten R² und R³ und R⁴ und R⁵ mit den an den Benzolkern angebundenen Teilstrukturen -O-CH₂-NR⁷-CH₂- und -O-CH₂-NR⁸-CH₂- einen zweiten und einen dritten Tetrahydrooxazin-Ring ausbilden,
wobei R⁷ und R⁸ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können,
mit der Maßgabe, dass mindestens einer der Substituenten R¹, R⁷ oder R⁸ 20 bis 3000 Kohlenstoffatomen aufweist und die übrigen Substituenten aus der Gruppe R¹, R², R³, R⁴, R⁵, R⁷ und R⁸, wenn sie für Hydrocarbylreste stehen, jeweils 1 bis 20 Kohlenstoffatome aufweisen.

Tetrahydrobenzoxazine sind im Prinzip als Zusatzstoffe für Kraftstoff- und Schmierstoffzusammensetzungen bekannt. So offenbaren die WO 01/25293 (3) und die WO 01/25294 (4) Tetrahydrobenzoxazine mit längerkettigen Resten wie Polyisobutenylresten, welche als Substituenten am Benzolkern sitzen, als ventilreinigende und ventilreinhaltende Ottokraftstoffdetergentien. Diese Tetrahydrobenzoxazine werden gemäß den in (3) und (4) genannten Herstellverfahren als Gemische mit den entsprechenden offenkettigen Mannich-Addukten des zugrundeliegenden Phenols erhalten und auch so in den Ottokraftstoffen eingesetzt.

Die Herstellung von Tetrahydrobenzoxazinen mit kurzkettigen Substituenten, welche sich als Pflanzenschutzmittel eignen, aus 4-Alkylphenolen und einem Addukt aus beispielsweise 1 Mol Cyclohexylamin und 2 Mol Formaldehyd oder Paraformaldehyd in Methanol oder Ethanol als Lösungsmittel ist in der US-A 2 806 031 (5) und der US-A 3 132 960 (6) beschrieben.

Die strukturelle Besonderheit der erfindungsgemäßen Tetrahydrobenzoxazine ist, dass sie mindestens einen längerkettigen Hydrocarbylrest mit bis zu 3000 Kohlenstoffatomen als einen der Substituenten R¹, R⁷ oder R⁸ an einem Oxazinring enthalten. In einer Ausführungsform ist dieser längerkettige Hydrocarbylrest ein Polyisobutenylrest. Dieser längerkettige Hydrocarbylrest, der vorzugsweise ein Polyisobutenylrest, umfasst vorzugsweise 16 bis 3000, insbesondere 20 bis 1000, vor allem 25 bis 500, ganz besonders bevorzugt 30 bis 250 Kohlenstoffatome. Im Falle von Polyisobutenylresten weisen diese zahlenmittlere Molekulargewichte Mₙ von 200 bis 40.000, vorzugsweise 500 bis 15.000, insbesondere 700 bis 7000, vor allem 900 bis 3000, ganz besonders bevorzugt 900 bis 1100 auf.

Der genannte längerkettige Hydrocarbylrest mit bis zu 3000 Kohlenstoffatomen kann in den Tetrahydrobenzoxazinen auch mehrfach, beispielsweise zweifach oder dreifach, vertreten sein.

In einer bevorzugten Ausführungsform treten ein oder zwei Polyisobutenyl-Reste mit einem zahlenmittleren Molekulargewicht Mₙ von 200 bis 40.000 im Molekül als Substituent R¹ und/oder R⁷ und/oder R⁸ auf.

Die übrigen Substituenten aus der Gruppe R¹, R², R³, R⁴, R⁵, R⁷ und R⁸, die nicht für längerkettigen Substituenten mit bis zu 3000 Kohlenstoffatomen oder für Polyisobutenylreste mit einem zahlenmittleren Molekulargewichte Mₙ von 200 bis 40.000 stehen, bezeichnen unabhängig voneinander Wasserstoffatome, Hydroxylgruppen oder, wenn sie für Hydrocarbylreste stehen, meist kürzerkettige Hydrocarbylreste mit 1 bis 20, vorzugsweise 1 bis 12, vor allem 1 bis 8, ganz besonders bevorzugt lineare oder verzweigte C₁- bis C₄-Al-kylreste. Typische Beispiele für die letzteren sind Methyl, Ethyl, n-Propyl, iso-Propyl, n-Butyl, 2-Butyl, sec.-Butyl und tert.-Butyl. Methylreste und tert.-Butylreste werden hierbei ganz besonders bevorzugt.

Bevorzugte erfindungsgemäße Tetrahydrobenzoxazine sind auch solche, bei denen die Substituenten R² und/oder R⁴, wenn sie für kürzerkettige Hydrocarbylreste stehen, lineare oder verzweigte C₁- bis C₄-Alkylreste, insbesondere Methylreste und/oder tert.-Butylreste, bezeichnen. Derartige Substitutionsmuster kommen natürlich nur für Tetrahydrobenzoxazine I mit insgesamt einem oder zwei Tetrahydrooxazin-Ringsystemen in Betracht.

Im Rest der Formel Y bezeichnet der Substituent X ein Kohlenwasserstoff-Brückenglied, welches aus einem oder mehreren, vorzugsweise 4 bis 800, insbesondere 10 bis 300, vor allem 12 bis 100 Isobuten-Einheiten besteht oder ein oder mehrere, vorzugsweise 4 bis 800, insbesondere 10 bis 300, vor allem 12 bis 100 Isobuten-Einheiten enthält. Besteht X aus Isobuten-Einheiten, erfolgt die Verknüpfung in der Relgel über die das α- und das ω-Kohlenstoffatom. Enthält X weitere Kohlenwasserstoff-Baueinheiten, sind dies vorzugsweise mittenständig angeordnete Initiatormolekül-Baueinheiten wie aromatische Ringsysteme, beispielsweise o-, m- oder p-Phenylen-Einheiten, und/oder Kohlenwasserstoff-Baueinheiten mit funktionellen Gruppen zur Verknüpfung, beispielsweise o-, m- oder p-Hydroxyphenyl-Gruppen, als beidseitiger Kettenabschluß. Derartige den Substituenten X zugrundeliegende telechele Polyisobuten-Systeme und ihre Herstellung werden beispielsweise in der US-A 4 429 099 (7) beschrieben.

Im Rest der Formel Z bzw. Z' bezeichnen die Substituenten R¹⁰ und R¹¹ vorzugsweise Wasserstoff und/oder lineare oder verzweigte C₁- bis C₄-Alkylreste, insbesondere Methylreste. Die Verbindung I mit einem Rest Z bzw. Z', bei dem R¹⁰ = R¹¹ = Methyl ist, leitet sich von Bisphenol A [2,2-Bis-(4-hydroxyphenyl)propan] ab. Herstellungsbedingt können Verbindungen I mit einem Rest Z und Verbindungen I mit dem entsprechenden Rest Z' auch als Mischungen vorliegen.

Unter Hydrocarbylresten mit bis zu 3000 Kohlenstoffatomen für die Substituenten R¹, R⁷ und R⁸ sollen hier reine Kohlenwasserstoffreste jeglicher Struktur verstanden werden, die definitionsgemäß auch noch durch ein oder mehrere Heteroatome aus der Gruppe O und S und/oder durch eine oder mehrere Gruppierungen NR⁶ unterbrochen sein können. Insbesondere sind Hydrocarbylreste Alkyl-, Alkenyl-, Cycloalkyl-, Aryl-, Alkylaryl-, Alkenylaryl- oder Arylalkyl-Reste.

Bei Unterbrechungen des Hydrocarbylrestes durch Gruppierungen NR⁶ sind auch solche Reste gemeint, bei denen am Ende die Gruppierungen NR⁶ formal in eine C-H-Bindung insertiert ist, also beispielsweise Substituenten R¹, R², R³, R⁴, R⁵, R⁷ oder R⁸ mit einer NH₂-Endgruppe. Derartige Hydrocarbylreste leiten sich z.B. von Polyaminen wie Ethylendiamin, Diethylentriamin, Triethylentetramin, Tetraethylenpentamin, etc. ab, bei denen eines der endständigen Stickstoffatome das N-Atom im Oxazin-Ring darstellt.

Der Ausdruck "Alkyl" umfasst geradkettige und verzweigte Alkylgruppen. Beispiele für Alkylgruppen sind neben den bereits oben genannten Methyl-, Ethyl-, n-Propyl-, iso-Propyl-, n-Butyl-, 2-Butyl-, sec.-Butyl- und tert.-Butyl-Resten insbesondere auch n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, n-Nonyl, n-Decyl, n-Dodecyl, n-Tridecyl, iso-Tridecyl, n-Tetradecyl (Myristyl), n-Hexadecyl (Palmityl), n-Octadecyl (Stearyl), und n-Eicosyl.

Beispiele für Alkenylreste sind Vinyl, 1-Propenyl, 2-Propenyl, Oleyl, Linolyl und Linolenyl.

Beispiele für Cylcoalkylreste sind C₅- bis C₇-Cycloalkylgruppen wie Cyclopentyl, Cyclohexyl und Cycloheptyl, welche noch durch Alkylgruppen, beispielsweise Methylreste, substituiert sein können.

Der Ausdruck "Aryl" umfasst einkernige, zweikernige, dreikernige und höherkernige aromatische Kohlenwasserstoffreste. Diese Arylreste können im Falle einer Substitution durch die beispielsweise vorgenannten Alkyl- und/oder Alkenylreste zu Alkylaryl- bzw. Alkenylarylresten noch 1, 2, 3, 4 oder 5, vorzugsweise 1, 2 oder 3 Substituenten tragen. Typische Beispiele sind Phenyl, Tolyl, Xylyl, Mesityl, Naphthyl, Fluorenyl, Anthracenyl, Phenanthrenyl, Naphthacenyl und Styryl. Ein typisches Beispiel für einen Arylalkylrest ist Benzyl.

Ist der längerkettige Hydrocarbylrest mit bis zu 3000 Kohlenstoffatomen ein Polyisobutenylrest, kann er auf prinzipiell jedem gängigen und kommerziell erhältlichen Polyisobuten basieren, welches in geeigneter Weise in die Synthese der Tetrahydrobenzoxazine I eingebracht wird. Ein solches Polyisobuten besitzt vorzugsweise ein zahlenmittleres Molekulargewicht Mₙ von mindestens 200. Bevorzugt sind Polyisobutene mit einem zahlenmittleren Molekulargewicht Mₙ im Bereich von 200 bis 40.000, besonders bevorzugt von 500 bis 15.000, vor allem von 700 bis 7000, insbesondere von 900 bis 3000 und ganz besonders bevorzugt von 900 bis 1100. Unter den Begriff "Polyisobuten" fallen im Sinne der vorliegenden Erfindung auch oligomere Isobutene wie dimeres, trimeres, tetrameres, pentameres, hexameres und heptameres Isobuten.

Vorzugsweise leiten sich die in den erfindungsgemäß verwendeten Tetrahydrobenzoxazine I eingebauten Polyisobutenylreste von sogenanntem "hochreaktiven" Polyisobuten ab. "Hochreaktive" Polyisobutene unterscheiden sich von den "niedrigreaktiven" Polyisobutenen durch den Gehalt an terminal angeordneten Doppelbindungen. So enthalten hochreaktive Polyisobutene wenigstens 50 Mol-% terminal angeordnete Doppelbindungen, bezogen auf die Gesamtanzahl an Polyisobuten-Makromolekülen. Besonders bevorzugt sind Polyisobutene mit wenigstens 60 Mol-% und insbesondere mit wenigstens 80 Mol-% terminal angeordnete Doppelbindungen, bezogen auf die Gesamtanzahl an Polyisobuten-Makromolekülen. Bei den terminal angeordneten Doppelbindungen kann es sich sowohl um Vinyldoppelbindungen [-CH=C(CH₃)₂] (β-Olefin) als auch um Vinyliden-Doppelbindungen [-CH-C(=CH₂)-CH₃] (α-Olefin) handeln. Außerdem weisen die im Wesentlichen homopolymere Polyisobutenylreste einheitliche Polymergerüste auf. Darunter werden im Rahmen der vorliegenden Erfindung solche Polyisobuten-Systeme verstanden, die zu wenigstens 85 Gew.-%, vorzugsweise zu wenigstens 90 Gew.-% und besonders bevorzugt zu wenigstens 95 Gew.-% aus Isobuteneinheiten der Wiederholungeinheit [-CH₂C(CH₃)₂-] aufgebaut sind.

Ein weiteres bevorzugtes Merkmal der Polyisobutene, die den erfindungsgemäßen Tetrahydrobenzoxazinen zugrunde liegen können, ist, dass sie zu wenigstens 15 Gew.-%, insbesondere zu wenigstens 50 Gew.-%, vor allem zu wenigstens 80 Gew.-% mit einer tert.-Butylgruppe [-CH₂C(CH₃)₃] terminiert sind.

Weiterhin weisen die als Basis für die erfindungsgemäßen Tetrahydrobenzoxazine vorzugsweise dienenden Polyisobutene vorzugsweise einen Polydispersitätsindex (PDI) von 1,05 bis 10, vorzugsweise von 1,05 bis 3,0, insbesondere von 1,05 bis 2,0 auf. Unter Polydispersität versteht man den Quotienten aus gewichtsmittlerem Molekulargewicht M_{w} und zahlenmittlerem Molekulargewicht Mₙ (PDI = M_{w}/Mₙ).

Unter als Basis für die erfindungsgemäßen Tetrahydrobenzoxazine vorzugsweise dienenden Polyisobutenen werden im Sinne der vorliegenden Erfindungen auch alle durch kationische Polymerisation erhältlichen Polymerisate verstanden, die vorzugsweise wenigstens 60 Gew.-% Isobuten, besonders bevorzugt wenigstens 80 Gew.-%, vor allem wenigstens 90 Gew.-% und insbesondere wenigstens 95 Gew.-% Isobuten einpolymerisiert enthalten. Daneben können die Polyisobutene weitere Buten-isomere wie 1- oder 2-Buten sowie davon verschiedene olefinisch ungesättigte Monomere, die mit Isobuten unter kationischen Polymerisationsbedingungen copolymerisierbar sind, einpolymerisiert enthalten.

Als Isobuten-Einsatzstoffe für die Herstellung von Polyisobutenen, die als Basis für die erfindungsgemäßen Tetrahydrobenzoxazine dienen können, eignen sich dementsprechend sowohl Isobuten selbst als auch isobutenhaltige C₄-Kohlenwasserstoffströme, beispielsweise C₄-Raffinate, C₄-Schnitte aus der Isobuten-Dehydrierung, C₄-Schnitte aus Steamcrackern, FCC-Crackern (FCC: Fluid Catalyzed Cracking), sofern sie weitgehend von darin enthaltenem 1,3-Butadien befreit sind. Besonders geeignete C₄-Kohlenwasserstoffströme enthalten in der Regel weniger als 500 ppm, vorzugsweise weniger als 200 ppm Butadien. Bei Einsatz von C₄-Schnitten als Einsatzmaterial übernehmen die von Isobuten verschiedenen Kohlenwasserstoffe die Rolle eines inerten Lösungsmittels.

Als mit Isobuten copolymerisierbare Monomere kommen Vinylaromaten wie Styrol und α-Methylstyrol, C₁-C₄-Alkylstyrole wie 2-, 3- und 4-Methylstyrol, sowie 4-tert.-Butylstyrol, Isoolefine mit 5 bis 10 C-Atomen wie 2-Methylbuten-1, 2-Methylpenten-1, 2-Methylhexen-1, 2-Ethylpenten-1, 2-Ethylhexen-1 und 2-Propylhepten-1 in Betracht.

Typische Polyisobutene, die als Basis für die erfindungsgemäßen Tetrahydrobenzoxazine dienen können, sind beispielsweise die Glissopal®-Marken der BASF Aktiengesellschaft, z. B. Glissopal 550, Glissopal 1000 und Glissopal 2300, sowie die Oppanol®-Marken der BASF Aktiengesellschaft, z.B. Oppanol B10, B12 und B15.

Bespiele für im Sinne der vorliegenden Erfindung typische Tetrahydrobenzoxazine mit einem Tetrahydrooxazin-Ring am Benzolkern sind die folgenden, wobei "PIB" einen von einem hochreaktiven Polyisobuten (Mₙ 1000) abgeleiteten Polyisobutenylrest und "PIB*" ein von einem hochreaktiven Polyisobuten (Mₙ 870) abgeleitetes Polyisobutenylen-Brückenglied bezeichnet:

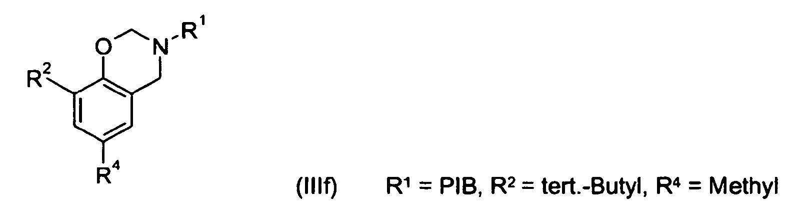

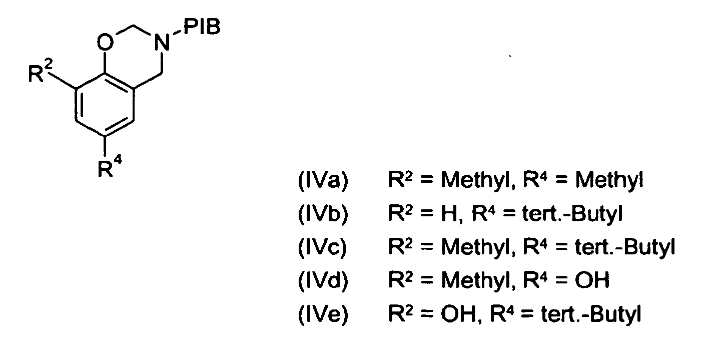

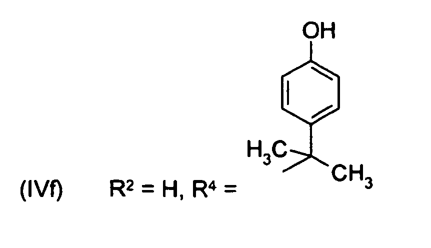

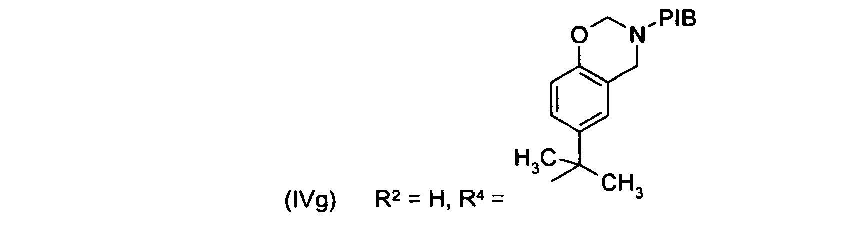

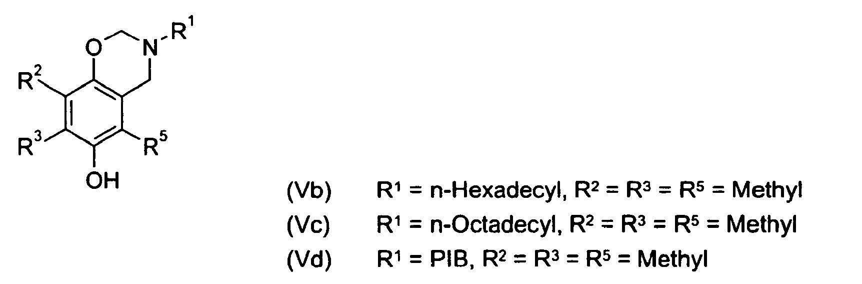

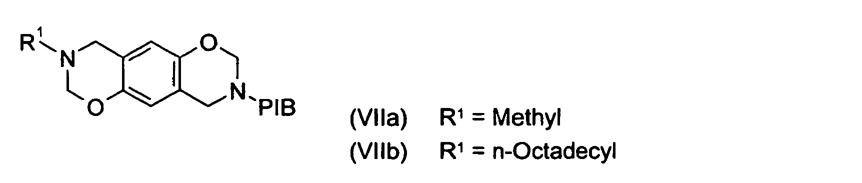

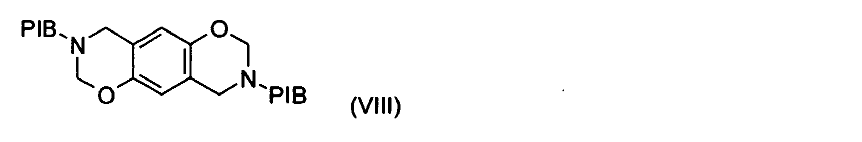

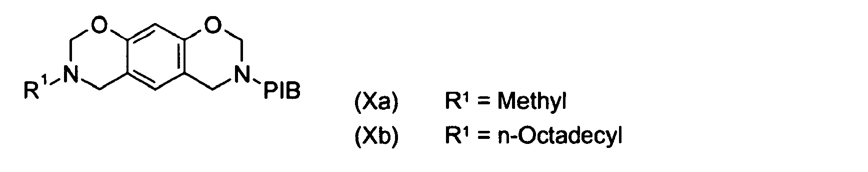

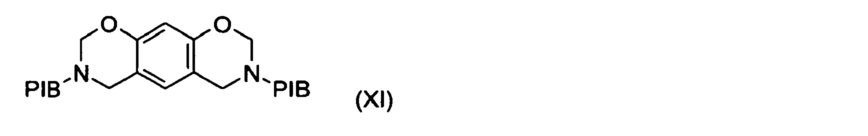

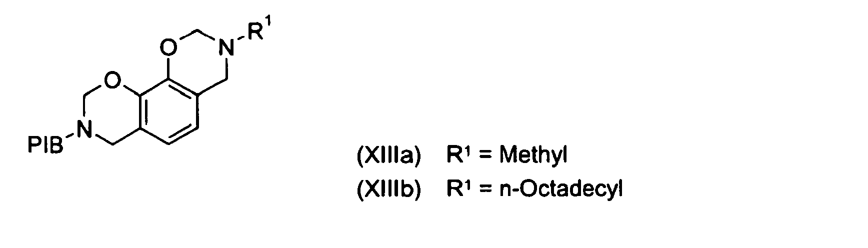

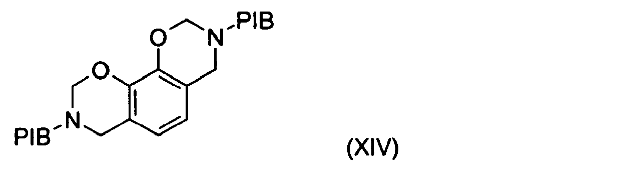

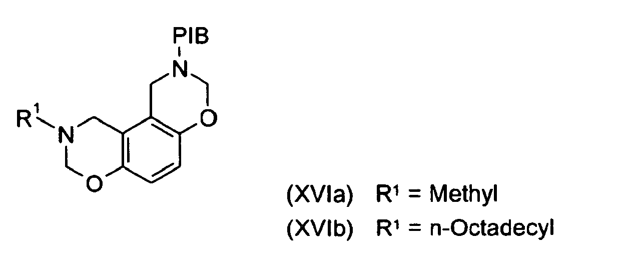

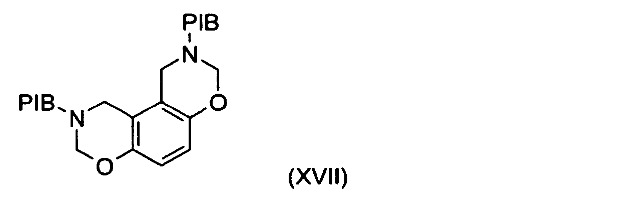

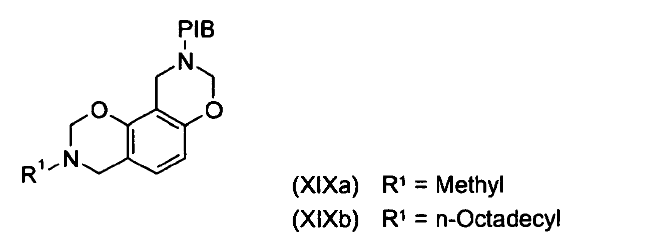

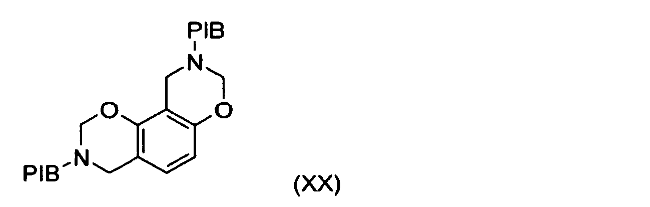

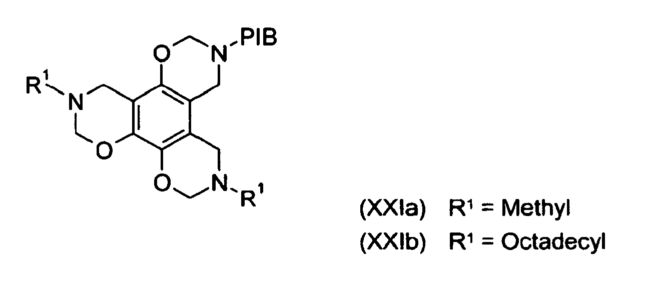

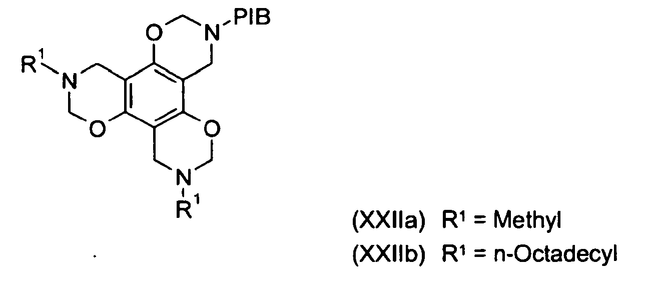

Herstellungsbedingt können auch Mischungen jeweils aus den Verbindungen Vlla + XVIa, VIIb + XVIb, VIII + XVII, Xa + XIXa, Xb + XIXb oder XI + XX auftreten und in dieser Form verwendet werden.

In einer bevorzugten Ausführungsform verwendet man Tetrahydrobenzoxazine, bei denen die Substituenten R³ und R⁴ oder R⁴ und R⁵ mit einer über den Substituenten R⁴ sauerstoff-angebundenen Teilstruktur -O-CH₂-NR⁷-CH₂- einen zweiten Tetrahydrooxazin-Ring ausbilden. Beispiele hierfür sind die oben aufgeführten Verbindungen VII bis XX.

Die beschriebenen Tetrahydrobenzoxazine werden als Stabilisatoren zur Stabilisierung von unbelebtem organischen Material gegen die Einwirkung von Licht, Sauerstoff und Wärme verwendet. Hierunter ist insbesondere ihre Wirkungsweise als Antioxidantien im herkömmlichen Sinne zu verstehen. Dazu werden diese Verbindungen in das zu stabilisierende Material während oder nach dessen Herstellung eingearbeitet und möglichst homogen verteilt. Die Konzentration dieser Verbindungen in dem zu stabilisierenden organischen Material beträgt in der Regel 0,0001 bis 5 Gew.-%, vorzugsweise 0,001 bis 5 Gew.-%, vor allem 0,01 bis 2 Gew.-%, insbesondere 0,05 bis 1 Gew.-%, bezogen auf das organische Material.

Unter unbelebtem organischen Material sind beispielsweise kosmetische Präparate wie Salben und Lotionen, Arzneimittelformulierungen wie Pillen und Zäpfchen, photographische Aufzeichnungsmaterialien, insbesondere photographische Emulsionen, Anstrichmittel und Kunststoffe zu verstehen. Dazu zählen weiterhin und insbesondere Mineralölprodukte und Kraftstoffe, z.B. Dieselkraftstoff, Ottokraftstoff, Turbinenkraftstoff, Motor- oder Schmieröle, Getriebeöle und Schmierfette.

Als Kunststoffe, die durch die beschriebenen Tetrahydrobenzoxazine stabilisiert werden können, seien beispielsweise genannt:
Polymere von Mono- oder Diolefinen, wie Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Polystyrol sowie Copolymere von Styrol oder α-Methylstyrol mit Dienen und/oder Acrylderivaten, z.B. Styrol-Butadien, Styrol-Acrylnitril (SAN), Styrol-Ethylmethacrylat, Styrol-Butadien-Ethylacrylat, Styrol-Acrylnitril-Methacrylat, Acrylnitril-Butadien-Styrol (ABS) oder Methylmethacrylat-Butadien-Styrol (MBS); halogenhaltige Polymere, z.B. Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acylderivaten oder Acetalen ableiten, z.B. Polyvinylalkohol und Polyvinylacetat;
Polyurethane, insbesondere thermoplastische Polyurethane, Polyamide, Polyharnstoffe, Polyphenylenether, Polyester, Polycarbonate, Polysulfone, Polyethersulfone und Polyetherketone.

Zu den Anstrichmitteln, die mit den beschriebenen Tetrahydrobenzoxazinen stabilisiert werden können, zählen unter anderem Lacke wie Alkydharzlacke, Dispersionslacke, Epoxydharzlacke, Polyurethanlacke, Acrylharzlacke und Cellulosenitratlacke, oder Lasuren wie Holzschutzlasuren.

Die beschriebenen Tetrahydrobenzoxazine eignen sich in besonders vorteilhafter Weise als Stabilisatoren in Turbinenkraftstoffen (jet fuels). Hierunter ist auch ihre Wirkungsweise als Antioxidantien im herkömmlichen Sinne zu verstehen. Insbesondere dienen sie über ihre Wirkungsweise als Stabilisatoren zur Verbesserung der thermischen Stabilität von Turbinenkraftstoffen. Weiterhin verhindern sie insbesondere auch über ihre Wirkungsweise als Stabilisatoren, d.h. in ihrer Eigenschaft als Dispergatoren, Ablagerungen im Kraftstoffsystem und/oder Verbrennungssystem von Turbinen. Turbinenkraftstoff werden vor allem zum Betreiben von Flugzeugturbinen eingesetzt.

Die beschriebenen Tetrahydrobenzoxazine lassen sich dadurch herstellen, dass man ein entsprechend substituiertes Phenol, Brenzcatechin, Resorcin, Hydrochinon, Phloroglucin oder Hydroxyhydrochinon mit einem aus einem Mol eines primären Amins der Formel R¹-NH₂ bzw. R⁷-NH₂ bzw. R⁸-NH₂, zwei Mol Formaldehyd oder einer Formaldehyd-freisetzenden Substanz und zwei Mol eines linearen oder verzweigten C₁- bis C₈-Al-kohols der Formel R⁹-OH vorgebildeten Reagenzes der allgemeinen Formel II 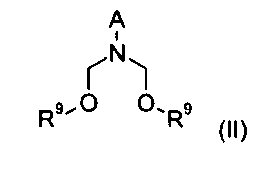 in der A für den Substituenten R¹, R⁷ oder R⁸, wobei diese die oben genannten Bedeutungen haben, steht, unter geeigneten Bedingungen umsetzt. Je nach gewünschter Anzahl der Tetrahydrooxazin-Ringe und der vorhandenen Hydroxylgruppen am Benzolkern kann hierbei die aromatische Hydroxylverbindung mit 1, 2 oder 3 Äquivalenten des Reagenzes II umgesetzt werden. Dabei weist das resultierende Tetrahydrobenzoxazin I- wie oben beschrieben - wenigstens einen Hydrocarbylrest mit bis zu 3000 Kohlenstoffatomen, welcher durch ein oder mehrere Heteroatome aus der Gruppe O und S und/oder durch eine oder mehrere Gruppierungen NR⁶ unterbrochen sein kann, auf.

Als lineare oder verzweigte C₁- bis C₈-Alkohole der Formel R⁹-OH eignen sich insbesondere lineare oder verzweigte C₁- bis C₈-Alkanole wie Methanol, Ethanol, Isopropanol, Isobutanol und sek.-Butanol.

Geeignete Bedingungen für die Umsetzung der aromatischen Hydroxyverbindungen mit dem Reagenz II sind beispielsweise Temperaturen von 0 bis 130°C, insbesondere 20 bis 100°C. Man arbeitet zweckmäßigerweise in einem inerten organischen Lösungsmittel, insbesondere einem aromatischen Kohlenwasserstoff wie Toluol oder Xylol.

Das Reagenz II wird üblicherweise aus den angegebenen Einsatzstoffen durch Abdestillation des bei seiner Bildung freiwerdenden Wassers hergestellt. Hierzu eignet sich beispielsweise das Erwärmen in einem wasserabscheidenden inerten organischem Solvens, insbesondere einem aromatischen Kohlenwasserstoff wie Toluol oder Xylol. Das freiwerdende Wasser kann im Prinzip jedoch auch im Reagenz II verbleiben, wenn es die weiteren Umsetzungen nicht stört. Als Formaldehyd-freisetzenden Substanz kann beispielsweise Paraformaldehyd verwendet werden.

Das genannte Herstellungsverfahren hat den Vorteil, dass das gewünschte Tetrahydrobenzoxazin in hoher Reinheit, d.h. weitgehend ohne unerwünschte Nebenprodukte oder zumindest in stark angereicherter Form, anfällt.

In einigen Fällen kann das gewünschte Tetrahydrobenzoxazin in entsprechender Reinheit auch ohne vorherige Bereitstellung des Reagenzes II durch direkte Umsetzung der entsprechend substituierten aromatischen Hydroxylverbindung mit einem Mol eines primären Amins der Formel R¹-NH₂ bzw. R⁷-NH₂ bzw. R⁸-NH₂ und zwei Mol Formaldehyd oder einer Formaldehyd-freisetzenden Substanz (pro umzusetzender Hydroxylgruppe) erhalten werden, wobei die Umsetzung gegebenenfalls in einem geeignetem Lösungsmittel wie einem Alkohol, beispielsweise einem linearen oder verzweigten C₁- bis C₈-Alkohol der Formel R⁹-OH, durchgeführt wird. Bei dieser Fahrweise besteht jedoch prinzipiell das Risiko, dass sich -je nach Eduktstruktur, Stöchiometrie und Umsetzungsbedingungen - nicht unerhebliche Mengen an nicht gewünschtem offenkettigem konventionellen Mannich-Addukt aus aromatischer Hydroxylverbindung, Amin und Formaldehyd bilden können.

Die Erfindung wird anhand der folgenden, nicht einschränkenden Beispiele näher erläutert.

### Herstellungsbeispiele

### Beispiel 1: Tetrahydrobenzoxazin der Formel Vc

In einem 500 ml-Vierhalskolben wurden 30 g Paraformaldehyd bei Raumtemperatur in 100 ml Isopropanol vorgelegt. 142 g aufgeschmolzenes n-Octadecylamin wurden rasch zugesetzt. Der Kolbeninhalt wurde bis zum Rückfluss erwärmt und 30 Minuten unter Rückfluss gerührt. 76 g Trimethylhydrochinon wurden in 3 Portionen innerhalb von 15 Minuten zugegeben. Es wurde 30 Minuten unter Rückfluss gerührt. Beim Abkühlen auf Raumtemperatur fiel ein Feststoff aus, der über eine D3-Filternutsche abgesaugt wurde. Der Filterrückstand wurde mehrmals mit Heptan gewaschen. Anschließend wurde der Rückstand über Nacht im Vakuumtrockenschrank bei 50°C und 100 mbar unter Stickstoffatmosphäre getrocknet. Man erhielt 186,4 g Produkt.
1H-NMR (400 MHz, 16 Scans, CDCl₃):
δ = 4,70 ppm, 2H, -CH₂-N(CH₃)-CH₂-O-
δ = 3,82 ppm, 2H, -CH₂-N(CH₃)-CH₂-O-

### Beispiel 2: Tetrahydrobenzoxazine der Formeln VIII + XVII

In einem 2000 ml-Vierhalskolben wurden 24 g Paraformaldehyd bei Raumtemperatur in 200 ml Isobutanol vorgelegt. 615 g Kerocom® PIBA (im Handel erhältliche 65 gew.-%ige Lösung von Polyisobutenylamin auf Basis von hochreaktivem Polyisobuten, Mn = 1000, in Mihagol, einem n-Alkan-Gemisch) wurden rasch zugesetzt. Der Kolbeninhalt wurde zum Rückfluss erwärmt und 30 Minuten unter Rückfluss gerührt. Dann setzte man 200 ml Toluol zu und schleppte das gebildete Wasser aus. 22 g Hydrochinon wurden in 200 ml n-Butanol gelöst innerhalb von 15 Minuten zugegeben. Anschließend wurde 60 Minuten unter Rückfluss gerührt. Die Lösung wurde bei 140°C und 5 mbar eingedampft. Man erhielt 440 g Produkt.
1H-NMR (400 MHz, 16 Scans, CDCl₃):
VIII: δ = 4,70 ppm, 2H, -CH₂-N(PIB)-CH₂-O-
   δ = 3,73 ppm, 2H, -CH₂-N(PIB)-CH₂-O-
XVII: δ = 4,75 ppm, 2H, CH₂-N(PIB)-CH₂-0O
   δ = 3,87 ppm, 2H, -CH₂-N(PIB)-CH₂-O-

Aus den Integralen der Spektren ergibt sich ein Gew.-Verhältnis von VIII : XVII = 1 : 1, 1.

### Beispiel 3: Tetrahydrobenzoxazin der Formeln Vd

In einem 4000 ml-Vierhalskolben wurden 52,8 g Paraformaldehyd bei Raumtemperatur in 400 ml Isopropanol vorgelegt. 1990 g Kerocom® PIBA (im Handel erhältliche 65 gew.-%ige Lösung von Polyisobutenylamin auf Basis von hochreaktivem Polyisobuten, Mn = 1000, in Mihagol, einem n-Alkan-Gemisch) wurden innerhalb von 78 Minuten zugesetzt. Der Kolbeninhalt wurde zum Rückfluss erwärmt und 40 Minuten unter Rückfluss gerührt. Danach wurden 138,1 g Trimethylhydrochinon bei 60 bis 80°C portionsweise zugegeben. Anschließend wurden 100 ml Toluol zugesetzt und es wurde 2 Stunden unter Rückfluß erwärmt. Nachdem nach Abkühlung auf Raumtemperatur die Isopropanol-Phase abgetrennt und verworden worden war, wurden 1000 ml Heptan zugesetzt. Die Lösung wurde mit Wasser und Methanol gewaschen und über Natriumsulfat getrocknet. Die trockene Lösung wurde bei 140°C und 5 mbar eingedampft. Man erhielt 1414 g Produkt.
1H-NMR (400 MHz, 16 Scans, CDCl₃):
δ = 4,70 ppm, 2H, -CH₂-N(PIB)-CH₂-O-
   δ = 3,81 ppm, 2H, -CH₂-N(PIB)-CH₂-O-
δ = 2,12, 2,07 und 2,00 ppm, jeweils 3H, -CH₃ am Aromaten

### Beispiel 4: Tetrahydrobenzoxazin der Formeln IIIf

In einem 2000 ml-Vierhalskolben wurden 24 g Paraformaldehyd bei Raumtemperatur in 150 ml Isopropanol vorgelegt. 800 g Kerocom® PIBA (im Handel erhältliche 65 gew.-%ige Lösung von Polyisobutenylamin auf Basis von hochreaktivem Polyisobuten, Mn = 1000, in Mihagol, einem n-Alkan-Gemisch) wurden innerhalb von 20 Minuten zugesetzt. Der Kolbeninhalt wurde zum Rückfluss erwärmt und 30 Minuten unter Rückfluss gerührt. Danach wurden 66 g 2-tert.-Butyl-4-methylphenol, gelöst in 150 ml Isopropanol, zugetropft. Anschließend wurde noch 2 Stunden unter Rückfluß erwärmt. Nachdem nach Abkühlung auf Raumtemperatur die Isopropanol-Phase abgetrennt und verworden worden war, wurde die Lösung bei 140°C und 5 mbar eingedampft. Man erhielt 574 g Produkt.
1H-NMR (400 MHz, 16 Scans, CDCl₃):
δ =4,80 ppm, 2H, -CH₂-N(PIB)-CH₂-O-
δ = 3,90 ppm, 2H, -CH₂-N(PIB)-CH₂-O-
δ = 2,21 ppm, 3H, -CH₃ am Aromaten

### Anwendungsbeispiele

### Beispiel 5: Überprüfung der thermischen Stabilität von Turbinenkraftstoff (jet fuel)

Es wurde ein Turbinenkraftstoff der Spezifikation Jet A nach ASTM D 1655 eingesetzt. Die Additivierung erfolgte mit jeweils 100 mg/l der Tetrahydrobenzoxazine bzw. Tetrahydrobenzoxazin-Gemische aus den Herstellungsbeispielen 2, 3 und 4.

In einem Dreihals-Glaskolben, der mit Rührer, Rückflusskühler und Thermometer versehen war, wurden zunächst bei Raumtemperatur 5 I Luft innerhalb 1 h durch 150 ml des zu untersuchenden Kraftstoffs geleitet. Anschließend wurde der Kraftstoff mit einem Ölbad auf 140 °C erhitzt und weitere 5 h bei dieser Temperatur gerührt. Nach Abkühlen auf Raumtemperatur wurde die gesamte Kraftstoffmenge über einen 0,45 µm Membranfilter filtriert. Anschließend wurde der Filterrückstand nach 45 min Trocknen im Trockenschrank bei 115 °C und anschließender 2-stündiger Trocknung unter Vakuum im Exsikkator gravimetrisch bestimmt:
Blindwert (ohne Additiv): 12,0 mg
erfindungsgemäß additiviert mit jeweils 100 mg/l der folgenden Verbindungen:

| | |
|---|---|
| Verbindungen der Formeln VIII + XVII (Herstellungsbeispiel 6): | 2,1 mg |
| Verbindung der Formel Vd (Herstellungsbeispiel 3): | 2,2 mg |
| Verbindung der Formel IIIf (Herstellungsbeispiel 4): | 1,0 mg |

Durch den Einsatz des erfindungsgemäßen Additivs konnte die durch thermische Belastung des Turbinenkraftstoffs entstehende Partikelmenge deutlich reduziert werden.

### Beispiel 6: Verbesserung der thermischen Stabilität von Turbinenkraftstoff (jet fuel)

Es wurde ein Turbinenkraftstoff in Anlehnung an die Spezifikation Jet A nach ASTM D 1655 eingesetzt. Die Überprüfung der Thermostabilität erfolgte nach der JFTOT-Breakpoint-Methode nach ASTM D 3241. Bei dem nicht additivierten Turbinenkraftstoff wurde ein Wert von 240°C ermittelt. Mit Kraftstoffen, die mit jeweils 100 mg/l eines nachfolgend aufgeführten erfindungsgemäß verwendeten Additivs additiviert waren, wurden die folgenden Werte gemessen:

### Verbindung der Formel Vd (Herstellungsbeispiel 3): 270°C

### Beispiel 7: Überprüfung der Wasserverträglichkeit von Turbinenkraftstoff

Es wurden ein Turbinenkraftstoff der Spezifikation Jet A-1 nach DEF STAN 91-91 verwendet.

Gemäß DIN 51415 bzw. ASTM D 1094 wurde nach Zugabe von 100 mg/l des Produktes aus Herstellungsbeispiel 2 die Wasserverträglichkeit des Turbinenkraftstoffes und damit die unerwünschte Neigung, Emulsionen zu bilden, bestimmt. Hierzu wurden 80 ml des additivierten Turbinenkraftstoffes und 20 ml Wasser in definierter Weise intensiv geschüttelt. Danach erfolgte eine visuelle Bewertung der Phasentrennschichten nach jeweils 1, 5, 30 und 60 Minuten. Bereits 5 Minuten nach Wasserzugabe erhielt man eine vollständige Trennung von Kraftstoff und Wasser, es blieben keine Emulsionsanteile zurück.

### Beispiel 8: Überprüfung der Wasserabtrennungseigenschaften von Turbinenkraftstoff

Es wurde ein Turbinenkraftstoff der Spezifikation Jet A-1 nach DEF STAN 91-91 eingesetzt. Die Überprüfung der Neigung von Turbinenkraftstoffen bezüglich ihrer Wasserabtrennungseigenschaften erfolgte nach ASTM D 3948 ("MSEP"-Test). Charakteristisch für diese Messungen ist die Verwendung eines Standard-Koaleszierfilters mit abschließender Trübungsmessung der Kraftstoffphase. Bei der Messung wurden die erfindungsgemäß verwendeten Additive in Kombination mit dem Antioxidans 2,6-Di-tert.-butyl-4-methylphenol ("BHT") und dem Metalldeaktivator N,N'-Disalicyliden-1,2-diaminopropan in einem hierfür üblichen Lösungsmittel geprüft. Die Dosierung der erfindungsgemäß verwendeten Additive betrug jeweils 215 mg/l (bezogen auf deren 100%igen Wirkstoffgehalt). Es wurden folgende Benotungen für das Trübungsverhalten ermittelt [relative Bewertungsskala von 0 (schlechteste Note) bis 100 (beste Note)]:

| | |
|---|---|
| Blindwert (ohne Additiv): | 99 |
| Verbindung der Formel Vd (Herstellungsbeispiel 3): | 94 |

Es traten keine Verschlechterungen im Vergleich mit nicht-additiviertem Turbinenkraftstoff auf.

## Patentansprüche

1. Tetrahydrobenzoxazine der allgemeinen Formel la 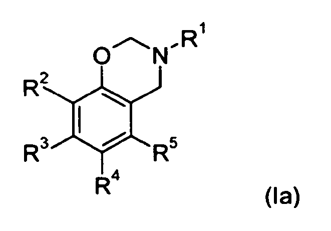 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und die Substituenten R², R³, R⁴ und R⁵ unabhängig voneinander für Wasserstoffatome oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können,
wobei der Substituent R⁴ auch für einen Rest der Formel Y stehen kann 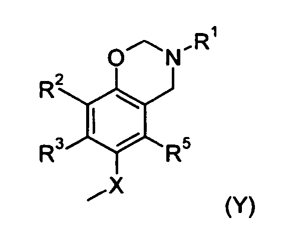 in dem die Substituenten R¹, R², R³ und R⁵ die vorgenannten Bedeutungen haben und der Substituent X ein Kohlenwasserstoff-Brückenglied bezeichnet, welches aus einem oder mehreren Isobuten-Einheiten besteht oder ein oder mehrere Isobuten-Einheiten enthält, oder
wobei der Substituent R⁴ auch für einen Rest der Formel Z oder Z' stehen kann 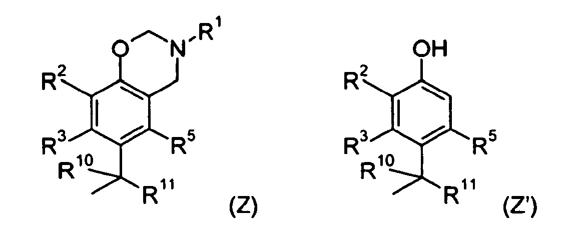 in denen die Substituenten R¹, R², R³ und R⁵ die vorgenannten Bedeutungen haben und die Substituenten R¹⁰ und R¹¹ gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR⁷-CH₂- auch einen zweiten Tetrahydrooxazin-Ring oder die Substituenten R² und R³ und R⁴ und R⁵ mit den an den Benzolkern angebundenen Teilstrukturen -O-CH₂-NR⁷-CH₂- und -O-CH₂-NR⁸-CH₂- auch einen zweiten und einen dritten Tetrahydrooxazin-Ring ausbilden können,
wobei R⁷ und R⁸ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können,
mit der Maßgabe, dass mindestens einer der Substituenten R¹ oder R⁷ oder R⁸ 25 bis 3000 Kohlenstoffatome aufweist und die übrigen Substituenten aus der Gruppe R¹, R², R³, R⁴, R⁵, R⁷ und R⁸, wenn sie für Hydrocarbylreste stehen, jeweils 1 bis 20 Kohlenstoffatome aufweisen.

2. Tetrahydrobenzoxazine der allgemeinen Formel Ib 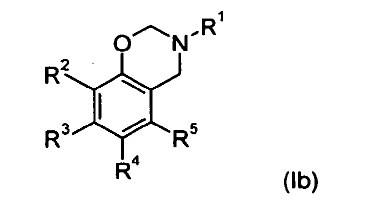 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und
die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können,
wobei der Substituent R⁴ auch für einen Rest der Formel Y stehen kann 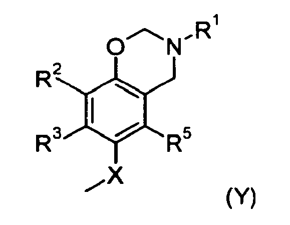 in dem die Substituenten R¹, R², R³ und R⁵ die vorgenannten Bedeutungen haben und der Substituent X ein Kohlenwasserstoff-Brückenglied bezeichnet, welches aus einem oder mehreren Isobuten-Einheiten besteht oder ein oder mehrere Isobuten-Einheiten enthält, oder
wobei der Substituent R⁴ auch für einen Rest der Formel Z oder Z' stehen kann 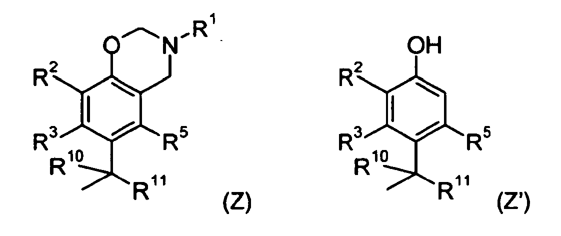 in denen die Substituenten R¹, R², R³ und R⁵ die vorgenannten Bedeutungen haben und die Substituenten R¹⁰ und R¹¹ gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR⁷-CH₂- auch einen zweiten Tetrahydrooxazin-Ring ausbilden können,
wobei R⁷ einen Hydrocarbylrest mit 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein kann,
mit der Maßgabe, dass mindestens einer der Substituenten R¹ oder R⁷ 16 bis 3000 Kohlenstoffatome aufweist und die übrigen Substituenten aus der Gruppe R¹, R², R³, R⁴, R⁵ und R⁷, wenn sie für Hydrocarbylreste stehen, jeweils 1 bis 20 Kohlenstoffatome aufweisen,
und mit der Maßgabe, dass die Substituenten R² und/oder R⁴ für Hydroxylgruppen stehen.

3. Tetrahydrobenzoxazine der allgemeinen Formel Id 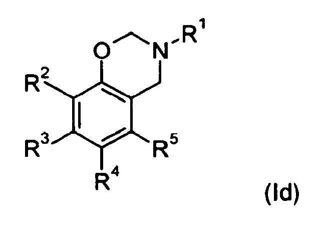 in der der Substituent R¹ einen Hydrocarbylrest mit 1 bis 3000 Kohlenstoffatomen, welcher durch ein oder mehrere Heteroatome aus der Gruppe O und S und/oder durch eine oder mehrere Gruppierungen NR⁶ unterbrochen sein kann, bezeichnet,
wobei R⁶ ein Wasserstoffatom oder einen C₁- bis C₄-Alkylrest bezeichnet, und die Substituenten R², R³, R⁴ und R⁵ unabhängig voneinander für Wasserstoffatome, Hydroxylgruppen oder Hydrocarbylreste mit jeweils 1 bis 3000 Kohlenstoffatomen stehen, welche durch ein oder mehrere Heteroatome aus der Gruppe O und S und/oder durch eine oder mehrere Gruppierungen NR⁶ unterbrochen sein können,
wobei der Substituent R⁴ auch für einen Rest der Formel Y stehen kann 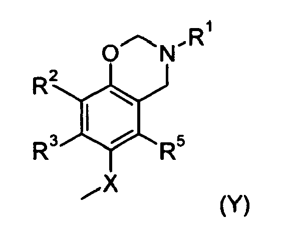 in dem die Substituenten R¹, R², R³ und R⁵ die vorgenannten Bedeutungen haben und der Substituent X ein Kohlenwasserstoff-Brückenglied bezeichnet, welches aus einem oder mehreren Isobuten-Einheiten besteht oder ein oder mehrere Isobuten-Einheiten enthält, oder
wobei der Substituent R⁴ auch für einen Rest der Formel Z stehen kann 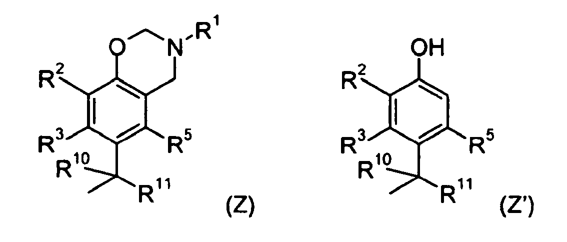 in denen die Substituenten R¹, R², R³ und R⁵ die vorgenannten Bedeutungen haben und die Substituenten R¹⁰ und R¹¹ gleich oder verschieden sein können und Wasserstoff oder einen C₁- bis C₁₀-Alkylrest bezeichnen,
und in der die Substituenten R² und R³ oder R³ und R⁴ oder R⁴ und R⁵ mit der an den Benzolkern angebundenen Teilstruktur -O-CH₂-NR⁷-CH₂- einen zweiten Tetrahydrooxazin-Ring oder die Substituenten R² und R³ und R⁴ und R⁵ mit den an den Benzolkern angebundenen Teilstrukturen -O-CH₂-NR⁷-CH₂- und -O-CH₂-NR⁸-CH₂- einen zweiten und einen dritten Tetrahydrooxazin-Ring ausbilden,
wobei R⁷ und R⁸ unabhängig voneinander Hydrocarbylreste mit jeweils 1 bis 3000 Kohlenstoffatomen bedeutet, welche durch ein oder mehrere Heteroatome aus der Gruppe O und S und/oder durch eine oder mehrere Gruppierungen NR⁶ unterbrochen sein können,
mit der Maßgabe, dass mindestens einer der Substituenten R¹, R⁷ oder R⁸ 20 bis 3000 Kohlenstoffatomen aufweist und die übrigen Substituenten aus der Gruppe R¹, R², R³, R⁴, R⁵, R⁷ und R⁸, wenn sie für Hydrocarbylreste stehen, jeweils 1 bis 20 Kohlenstoffatome aufweisen.

## Claims

1. A tetrahydrobenzoxazine of the general formula Ia 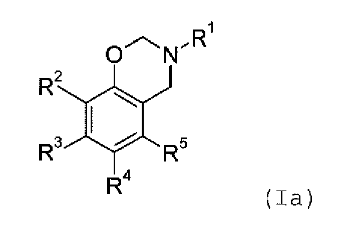 in which the substituent R¹ represents a hydrocarbyl radical which has from 1 to 3000 carbon atoms and may be interrupted by one or more heteroatoms from the group of O and S and/or by one or more NR⁶ moieties,
where R⁶ represents a hydrogen atom or a C₁- to C₄-alkyl radical, and
the substituents R², R³, R⁴ and R⁵ are each independently hydrogen atoms or hydrocarbyl radicals which have in each case from 1 to 3000 carbon atoms and may be interrupted by one or more heteroatoms from the group of O and S and/or by one or more NR⁶ moieties,
where the substituent R⁴ may also be a radical of the formula Y 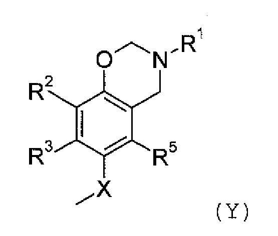 in which the substituents R¹, R², R³ and R⁵ are each as defined above and the substituent X is a hydrocarbon bridging element which consists of one or more isobutene units or comprises one or more isobutene units, or
where the substituent R⁴ may also be a radical of the formula Z or Z' 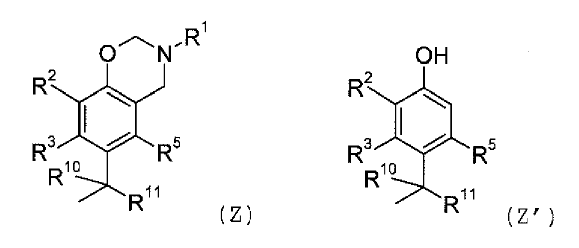 in which the substituents R¹, R², R³ and R⁵ are each as defined above and the substituents R¹⁰ and R¹¹ may be the same or different and represent hydrogen or a C₁- to C₁₀-alkyl radical,
and in which the substituents R² and R³ or R³ and R⁴ or R⁴ and R⁵, together with the part-structure -O-CH₂-NR⁷-CH₂- attached to the benzene ring, may also form a second tetrahydrooxazine ring, or the substituents R² and R³ and R⁴ and R⁵, together with the part-structures -O-CH₂-NR⁷-CH₂- and -O-CH₂-NR⁸-CH₂- attached to the benzene ring, may also form a second and a third tetrahydrooxazine ring,
where R⁷ and R⁸ are each independently hydrocarbyl radicals which have in each case from 1 to 3000 carbon atoms and may be interrupted by one or more heteroatoms from the group of 0 and S and/or by one or more NR⁶ moieties,
with the proviso that at least one of the substituents R¹ or R⁷ or R⁸ has from 25 to 3000 carbon atoms and the remaining substituents from the group of R¹, R², R³, R⁴, R⁵, R⁷ and R⁸, when they are hydrocarbyl radicals, each have from 1 to 20 carbon atoms.

2. A tetrahydrobenzoxazine of the general formula Ib 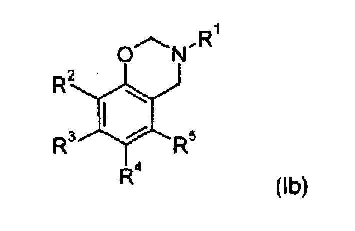 in which the substituents R¹ represents a hydrocarbyl radical which has from 1 to 3000 carbon atoms and may be interrupted by one or more heteroatoms from the group of O and S and/or by one or more NR⁶ moieties,
where R⁶ represents a hydrogen atom or a C₁- to C₄-alkyl radical, and
the substituents R², R³, R⁴ and R⁵ are each independently hydrogen atoms, hydroxyl groups or hydrocarbyl radicals which have in each case from 1 to 3000 carbon atoms and may be interrupted by one or more heteroatoms from the group of 0 and S and/or by one or more NR⁶ moieties,
where the substituent R⁴ may also be a radical of the formula Y 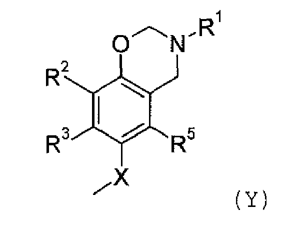 in which the substituents R¹, R², R³ and R⁵ are each as defined above and the substituent X is a hydrocarbon bridging element which consists of one or more isobutene units or comprises one or more isobutene units, or
where the substituent R⁴ may also be a radical of the formula Z or Z' 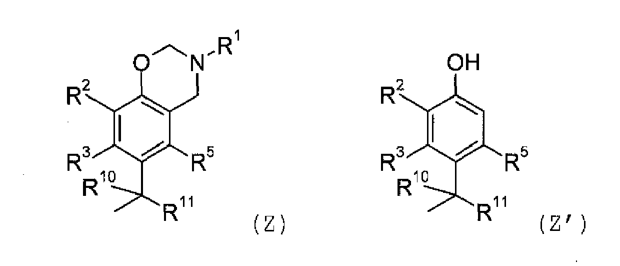 in which the substituents R¹, R², R³ and R⁵ are each as defined above and the substituents R¹⁰ and R¹¹ may be the same or different and represent hydrogen or a C₁- to C₁₀-alkyl radical,
and in which the substituents R² and R³ or R³ and R⁴ or R⁴ and R⁵, together with the part-structure -O-CH₂-NR⁷-CH₂- attached to the benzene ring, may also form a second tetrahydrooxazine ring,
where R⁷ is a hydrocarbyl radical which has from 1 to 3000 carbon atoms and may be interrupted by one or more heteroatoms from the group of O and S and/or by one or more NR⁶ moieties,
with the proviso that at least one of the substituents R¹ or R⁷ has from 16 to 3000 carbon atoms and the remaining substituents from the group of R¹, R², R³, R⁴, R⁵ and R⁷, when they are hydrocarbyl radicals, each have from 1 to 20 carbon atoms,
and with the proviso that the substituents R² and/or R⁴ are each hydroxyl groups.

3. A tetrahydrobenzoxazine of the general formula Id 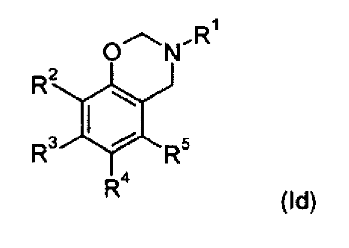 in which the substituent R¹ represents a hydrocarbyl radical which has from 1 to 3000 carbon atoms and may be interrupted by one or more heteroatoms from the group of 0 and S and/or by one or more NR⁶ moieties,
where R⁶ represents a hydrogen atom or a C₁- to C₄-alkyl radical, and
the substituents R², R³, R⁴ and R⁵ are each independently hydrogen atoms, hydroxyl groups or hydrocarbyl radicals which have in each case from 1 to 3000 carbon atoms and may be interrupted by one or more heteroatoms from the group of O and S and/or by one or more NR⁶ moieties,
where the substituent R⁴ may also be a radical of the formula Y 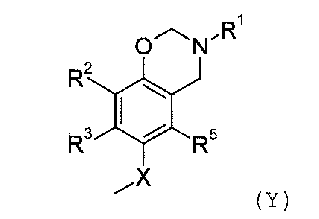 in which the substituents R¹, R², R³ and R⁵ are each as defined above and the substituent X is a hydrocarbon bridging element which consists of one or more isobutene units or comprises one or more isobutene units, or
where the substituent R⁴ may also be a radical of the formula Z or Z' 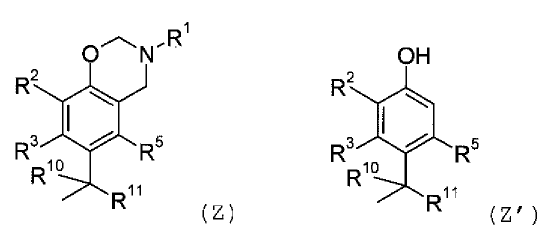 in which the substituents R¹, R², R³ and R⁵ are each as defined above and the substituents R¹⁰ and R¹² may be the same or different and represent hydrogen or a C₁- to C₁₀-alkyl radical,
and in which the substituents R² and R³ or R³ and R⁴ or R⁴ and R⁵, together with the part-structure -O-CH₂-NR⁷-CH₂- attached to the benzene ring, form a second tetrahydrooxazine ring, or the substituents R² and R³ and R⁴ and R⁵, together with the part-structures -O-CH₂-NR⁷- CH₂- and -O-CH₂-NR⁸-CH₂-attached to the benzene ring, form a second and a third tetrahydrooxazine ring,
where R⁷ and R⁸ are each independently hydrocarbyl radicals which have in each case from 1 to 3000 carbon atoms and may be interrupted by one or more heteroatoms from the group of O and S and/or by one or more NR⁶ moieties,
with the proviso that at least one of the substituents R¹, R⁷ or R⁸ has from 20 to 3000 carbon atoms or polyisobutenyl radicals 3000 carbon atoms and the remaining substituents from the group of R¹, R², R³, R⁴, R⁵, R⁷ and R⁸, when they are hydrocarbyl radicals, each have from 1 to 20 carbon atoms.

## Revendications

1. Tétrahydrobenzoxazines de formule générale la 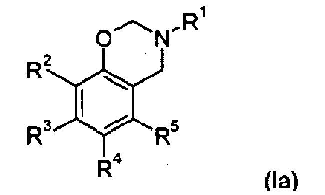 dans laquelle le substituant R¹ désigne un radical hydrocarbyle comprenant 1 à 3000 atomes de carbone, qui peut être interrompu par un ou plusieurs hétéroatomes du groupe O et S et/ou par un ou plusieurs groupements NR⁶,
R⁶ désignant un atome d'hydrogène ou un radical C₁-C₄-alkyle, et
les substituants R², R³ R⁴ et R⁵ représentant, indépendamment l'un de l'autre, des atomes d'hydrogène ou des radicaux hydrocarbyle comprenant à chaque fois 1 à 3000 atomes de carbone, qui peuvent être interrompus par un ou plusieurs hétéroatomes du groupe 0 et S et/ou par un ou plusieurs groupements NR⁶,
le substituant R⁴ pouvant également représenter un radical de formule Y 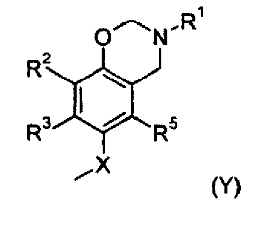 dans laquelle les substituants R¹, R², R³ et R⁵ présentent les significations susmentionnées et le substituant X désigne un élément de pont hydrocarboné, qui est constitué par une ou plusieurs unités isobutène ou qui contient une ou plusieurs unités isobutène, ou le substituant R⁴ pouvant également représenter un radical de formule Z ou Z' 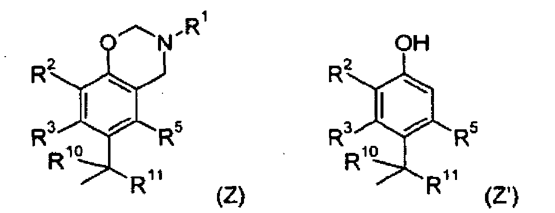 dans lesquelles les substituants R¹, R², R³ et R⁵ présentent les significations susmentionnées et les substituants R¹⁰ et R¹¹ peuvent être identiques ou différents et désignent hydrogène ou un radical C₁-C₁₀-alkyle,
et dans lesquelles les substituants R² et R³ ou R³ et R⁴ ou R⁴ et R⁵ peuvent également former, avec la structure partielle -O-CH₂-NR⁷-CH₂- liée au noyau de benzène, un deuxième cycle tétrahydrooxazine ou les substituants R² et R³ et R⁴ et R⁵ peuvent également former, avec les structures partielles -O-CH₂-NR⁷-CH₂- et -O-CH₂-NR⁸-CH₂-liées au noyau de benzène, un deuxième et un troisième cycle tétrahydrooxazine,
R⁷ et R⁸ représentant, indépendamment l'un de l'autre, des radicaux hydrocarbyle comprenant à chaque fois 1 à 3000 atomes de carbone, qui peuvent être interrompus par un ou plusieurs hétéroatomes du groupe O et S et/ou par un ou plusieurs groupements NR⁶,
à condition qu'au moins un des substituants R¹ ou R⁷ ou R⁸ présente 25 à 3000 atomes de carbone et les autres substituants du groupe R¹, R², R³, R⁴, R⁵, R⁷ et R⁸, lorsqu'ils représentent des radicaux hydrocarbyle, présentent à chaque fois 1 à 20 atomes de carbone.

2. Tétrahydrobenzoxazines de formule générale Ib 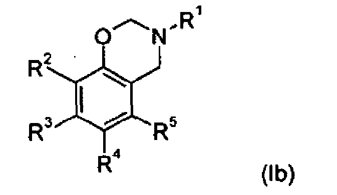 dans laquelle le substituant R¹ désigne un radical hydrocarbyle comprenant 1 à 3000 atomes de carbone, qui peut être interrompu par un ou plusieurs hétéroatomes du groupe 0 et S et/ou par un ou plusieurs groupements NR⁶,
R⁶ désignant un atome d'hydrogène ou un radical C₁-C₄-alkyle, et
et les substituants R², R³, R⁴ et R⁵ représentant, indépendamment l'un de l'autre, des atomes d'hydrogène, des groupes hydroxyle ou des radicaux hydrocarbyle comprenant à chaque fois 1 à 3000 atomes de carbone, qui peuvent être interrompus par un ou plusieurs hétéroatomes du groupe O et S et/ou par un ou plusieurs groupements NR⁶,
le substituant R⁴ pouvant également représenter un radical de formule Y 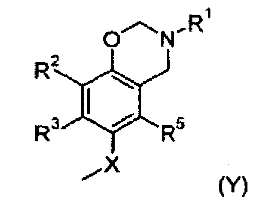 dans laquelle les substituants R¹, R², R³ et R⁵ présentent les significations susmentionnées et le substituant X désigne un élément de pont hydrocarboné, qui est constitué par une ou plusieurs unités isobutène ou qui contient une ou plusieurs unités isobutène, ou le substituant R⁴ pouvant également représenter un radical de formule Z ou Z' 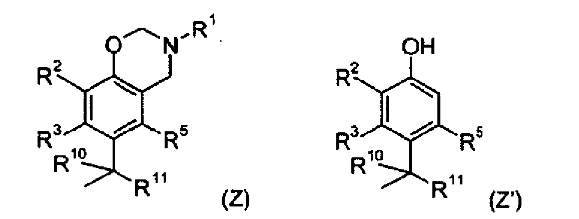 dans lesquelles les substituants R¹, R², R³ et R⁵ présentent les significations susmentionnées et les substituants R¹⁰ et R¹¹ peuvent être identiques ou différents et désignent hydrogène ou un radical C₁-C₁₀-alkyle,
et dans lesquelles les substituants R² et R³ ou R³ et R⁴ ou R⁴ et R⁵ peuvent également former, avec la structure partielle -O-CH₂-NR⁷-CH₂- liée au cycle benzène, un deuxième cycle tétrahydrooxazine,
R⁷ signifiant un radical hydrocarbyle comprenant 1 à 3000 atomes de carbone, qui peut être interrompu par un ou plusieurs hétéroatomes du groupe 0 et S et/ou par un ou plusieurs groupements NR⁶,
à condition qu'au moins un des substituants R¹ ou R⁷ présente 16 à 3000 atomes de carbone et les autres substituants du groupe R¹, R², R³, R⁴, R⁵ et R⁷, lorsqu'ils représentent des radicaux hydrocarbyle, présentent à chaque fois 1 à 20 atomes de carbone,
et à condition que les substituants R² et/ou R⁴ représentent des groupes hydroxyle.

3. Tétrahydrobenzoxazines de formule générale Id 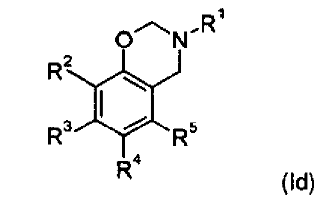 dans laquelle le substituant R¹ désigne un radical hydrocarbyle comprenant 1 à 3000 atomes de carbone, qui peut être interrompu par un ou plusieurs hétéroatomes du groupe O et S et/ou par un ou plusieurs groupements NR⁶,
R⁶ désignant un atome d'hydrogène ou un radical C₁-C₄-alkyle, et
les substituants R², R³, R⁴ et R⁵ représentant, indépendamment l'un de l'autre, des atomes d'hydrogène, des groupes hydroxyle ou des radicaux hydrocarbyle comprenant à chaque fois 1 à 3000 atomes de carbone, qui peuvent être interrompus par un ou plusieurs hétéroatomes du groupe 0 et S et/ou par un ou plusieurs groupements NR⁶
le substituant R⁴ pouvant également représenter un radical de formule Y 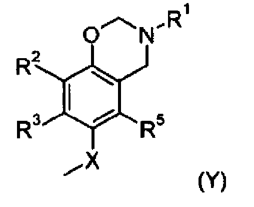 dans lesquelles les substituants R¹, R², R³ et R⁵ présentent les significations susmentionnées et le substituant X désigne un élément de pont hydrocarboné, qui est constitué par une ou plusieurs unités isobutène ou qui contient une ou plusieurs unités isobutène, ou le substituant R⁴ pouvant également représenter un radical de formule Z ou Z' 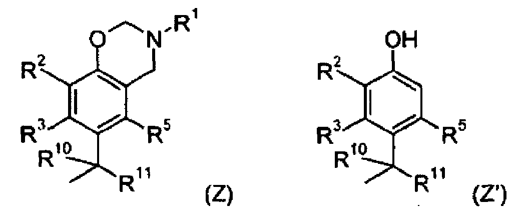 dans lesquelles les substituants R¹, R², R³ et R⁵ présentent les significations susmentionnées et les substituant R¹⁰ et R¹¹ peuvent être identiques ou différents et désignent hydrogène ou un radical C₁-C₁₀-alkyle,
et dans lesquelles les substituants R² et R³ ou R³ et R⁴ ou R⁴ et R⁵ peuvent également former, avec la structure partielle -O-CH₂-NR⁷-CH₂- liée au noyau de benzène, un deuxième cycle tétrahydrooxazine ou les substituants R² et R³ et R⁴ et R⁵ peuvent également former, avec les structures partielles -O-CH₂-NR⁷-CH₂- et -O-CH₂-NR⁸-CH₂-liées au noyau de benzène, un deuxième et un troisième cycle tétrahydrooxazine,
R⁷ et R⁸ représentant, indépendamment l'un de l'autre, des radicaux hydrocarbyle comprenant à chaque fois 1 à 3000 atomes de carbone, qui peuvent être interrompus par un ou plusieurs hétéroatomes du groupe O et S et/ou par un ou plusieurs groupements NR⁶,
à condition qu'au moins un des substituants R¹, R⁷ ou R⁸ présente 20 à 3000 atomes de carbone et les autres substituants du groupe R¹, R², R³, R⁴, R⁵, R⁷ et R⁸, lorsqu'ils représentent des radicaux hydrocarbyle, présentent à chaque fois 1 à 20 atomes de carbone.
